# EUROPEAN PATENT SPECIFICATION

(11) **EP 3 601 523 B1**
(45) Date of publication and mention of the grant of the patent: **22.02.2023**
(21) Application number: 18775026.0
(22) Date of filing: 27.03.2018
(51) Int. Cl.: C12N 1/06, C12N 1/21, C12N 15/10

(54) **METHOD FOR HIGH -THROUGHPUT GENOMIC DNA EXTRACTION**
VERFAHREN ZUR GENOMISCHEN DNA-EXTRAKTION MIT HOHEM DURCHSATZ
PROCÉDÉ D'EXTRACTION D'ADN GÉNOMIQUE À HAUT RENDEMENT

(30) Priority: 28.03.2017 US 201762477955 P
(43) Date of publication of application: 05.02.2020
(73) Proprietor: Ginkgo Bioworks, Inc., Boston, MA 02210 (US)
(72) Inventor: MURRAY, Jessica, Robyn, St. Louis, MO 63167 (US); VAUGHAN, Brian, St. Louis, MO 63167 (US)
(74) Representative: Paemen, Liesbet R.J.
(86) International application number: PCT/US2018/024425
(87) International publication number: WO 2018/183229

(56) References cited:
- US-A1- 2002 172 949
- US-A1- 2013 323 815
- CHRISTINA GILL ET AL: "Evaluation of Lysis Methods for the Extraction of Bacterial DNA for Analysis of the Vaginal Microbiota", PLOS ONE, vol. 11, no. 9, 19 September 2016 (2016-09-19), page e0163148, XP055556168, DOI: 10.1371/journal.pone.0163148
- MMP FARIA ET AL: "The development and application of a molecular community profiling strategy to identify polymicrobial bacterial DNA in the whole blood of septic patients", BMC MICROBIOLOGY, BIOMED CENTRAL LTD, GB, vol. 15, no. 1, 16 October 2015 (2015-10-16), page 215, XP021229126, ISSN: 1471-2180, DOI: 10.1186/S12866-015-0557-7
- SANQING YUAN ET AL: "Evaluation of Methods for the Extraction and Purification of DNA from the Human Microbiome", PLOS ONE, vol. 7, no. 3, 1 March 2012 (2012-03-01), pages 1-10, XP055249303, DOI: 10.1371/journal.pone.0033865

## Description

### REFERENCE TO RELATED APPLICATION

### FIELD OF THE INVENTION

The present invention generally relates to the extraction and isolation of genomic DNA from a broad range of microbial species.

### BACKGROUND

Each tablespoon of soil can contain up to a billion microbial cells. The ability to extract DNA from a greater number and more diverse collection of microbial isolates is critical to characterizing microbial species and developing microbial solutions for farmers. Just as microbes play a key role in human health, the microbial communities associated with crop plants can have significant beneficial effects on plant health and yield.

A significant hurdle in genomic and metagenomic analysis of microbes and microbial collections or communities is DNA extraction bias. If DNA cannot be extracted from a particular microbial isolate or strain then that isolate or strain may be underrepresented or absent in any subsequent DNA analysis of a microbial community comprising the recalcitrant isolate or strain. Thus, there remains a need to provide new and innovative solutions to effectively extract genomic DNA (gDNA) from microbial isolates or strains, including those from the environment, to further our understanding of soil/plant microbiomes and develop novel microorganisms for agricultural use.

### SUMMARY

In one aspect, methods are provided for extracting genomic DNA (gDNA) from a microbial sample comprising the steps of: a) subjecting the microbial sample to mechanical disruption; b) subsequent to step a, contacting the mechanically disrupted microbial sample with an enzyme cocktail comprising a proteinase and a glycoside hydrolase to produce a lysate of the microbial sample; and c) purifying microbial gDNA from the lysate of the microbial sample. In certain embodiments, said mechanical disruption comprises the use of beads, for example garnet beads, carbide beads, metal beads, glass beads, or ceramic beads. In some embodiments, the beads have a diameter between 0.1 mm and 3.0 mm or the beads have a diameter between 0.1 mm and 0.5 mm. Said mechanical disruption may further comprise the use of a shaking homogenizer. In certain embodiments, said microbial sample subjected to mechanical disruption is in a lysis buffer, and wherein the lysis buffer comprises a cationic detergent. In exemplary embodiments, the cationic detergent is cetyltrimethylammonium bromide. In some embodiments, the mechanically disrupted microbial sample is contacted with the enzyme cocktail for about 30 to 60 minutes or at a temperature between about 25°C and about 60°C. Contacting the mechanically disrupted microbial sample with an enzyme cocktail may comprise vortexing, for example vortexing at intervals of between about 5 and about 15 minutes. Contacting the mechanically disrupted microbial sample with an enzyme cocktail may comprise sonication, for example sonication at intervals of between about 5 and about 15 minutes.

In further embodiments, the proteinase may be a serine protease, for example proteinase K. The proteinase K may be at a concentration of between about 0.1 and about 1.0 mg/mL.

In yet further embodiments, the glycoside hydrolase may be a N-acetyl muramidase, N-acetyl muramide glycanhydrolase, or muramidase. The glycoside hydrolase may be a lysozyme. In some embodiments, said lysozyme may be at a concentration of between about 0.1 mg/mL and about 1.0 mg/mL.

In still some embodiments, said enzyme cocktail may further comprise a second glycoside hydrolase, wherein the glycoside hydrolase is different than the second glycoside hydrolase. The second glycoside hydrolase may be a N-acetyl muramidase, N-acetyl muramide glycanhydrolase, or muramidase. In further embodiments, the second glycoside hydrolase may be a chitinase or chitosanase. In yet further embodiments, the second glycoside hydrolase is mutanolysin. In certain examples, the mutanolysin is at a concentration of between about 10 U/mL and about 250 U/mL. In some embodiments, said enzyme cocktail comprises additional enzymes selected from the group consisting of RNase A, lysostephin, labiase, lysyl-endopeptidase, and a combination thereof. In certain examples, the enzyme cocktail comprises RNase A at a concentration of between about 10 mg/mL and about 100 mg/mL.

In some embodiments, purifying microbial gDNA from said lysate comprises: 1) adding a binding buffer comprising a chaotropic agent and a chelating agent to the lysate of the microbial sample to produce a binding mixture; 2) filtering the binding mixture through a DNA-binding matrix; 3) filtering a wash buffer, comprising an alcohol, through the matrix; and 4) filtering an elution buffer through the matrix to produce an eluate, wherein the eluate comprises the microbial gDNA. In certain embodiments, the chaotropic agent is guanidine hydrochloride and the chelating agent is ethylenediaminetetraacetic acid. In some examples, the DNA-binding matrix comprises a membrane, borosilicate glass, beads, resin, or any combination thereof.

In further embodiments, said microbial sample may comprise Gram-negative bacteria, Gram-positive bacteria, or a combination of both. In yet further embodiments, said microbial sample comprises environmental isolates.

### BRIEF DESCRIPTION OF THE DRAWINGS

**FIG. 1****.** Histogram demonstrating the varying lytic efficacy of cationic detergents on microbial gDNA extraction yield.
**FIG. 2****.** Histogram demonstrating the varying lytic efficacy of lysozyme on the microbial gDNA extraction yield of the disclosed method. (AG = Agradis method).
**FIG. 3****.** Histogram demonstrating the varying lytic efficacy of lysozyme on the number of microbial isolates extracted using the disclosed method. (AG = Agradis method).
**FIG. 4****.** Histogram demonstrating the varying lytic efficacy of lysozyme incubation time on microbial gDNA extraction yield using the disclosed method.
**FIG. 5****.** Histogram demonstrating the varying lytic efficacy of lysozyme concentration on microbial gDNA extraction yield using the disclosed method.
**FIG. 6****.** Histogram demonstrating the lytic efficacy of various microbial gDNA extraction methodologies as measured by the number of microbial isolates obtained.
**FIG. 7****.** Pairwise similarity plot comparing the similarity and diversity of the taxonomic profiles of microbial isolates with successfully extracted gDNA as a result of various microbial gDNA extraction methodologies.
**FIG. 8****.** Histogram demonstrating the cost efficiencies of various microbial gDNA extraction methodologies.

### DETAILED DESCRIPTION

Disclosed herein are novel methods for the extraction and isolation of DNA from a wide range of microorganisms, some of which may be recalcitrant to standard DNA extraction methodologies. The methods described herein can be used to assist in developing new microbial products for agriculture, by helping with the identification and characterization of microorganisms and microbial communities.

Microbial community profiling using nucleic acid analysis is important for understanding the complex microbiomes associated with plants and soil. High-throughput DNA sequencing has become an important tool for the identification of microbes associated with the soil, rhizosphere, endosphere, surfaces, etc., of crop plants, or other agricultural or non-agricultural environments. However, methods relying on DNA analysis and sequencing to identify or characterize microbial species or isolates is dependent on successfully extracting and isolating DNA from those microbial species or isolates. Current methodologies for extracting DNA from microbes involve mechanical, thermal, or enzymatic disruption of microbial cell walls. However, these methods still have difficulty in lysing the cell walls of many microbial species, such as the thick peptidoglycan cell walls of Gram-positive bacteria. Lysis of these microbes must be successful in disrupting their extensive cell wall to extract their DNA, and thus allow for their detection or quantification in a sample. Furthermore, the use of multiple separate extraction protocols for each microbial sample is time-consuming and impractical, and not amenable to high-throughput use or screening.

An aspect of the present disclosure provides a method of rapidly extracting DNA from a large diversity of microorganisms, including those normally resistant to lysis. The method comprises extracting and/or isolating DNA from microorganisms by mechanically disrupting cell walls and membranes, and then using a cocktail of enzymes to successfully lyse or further disrupt the microbes to make their DNA available for isolation or extraction. In an embodiment, a microbial sample or culture is first subjected to mechanical disruption, for example, through "bead beating" using movement of balls, beads or other particles. As used herein, the terms "bead" and "beads" refer to solid particle(s), bead(s), ball(s), etc., of suitable size that may be used for lysis of microbial cells by their movement within a tube, container, etc. Such beads will be sufficiently small to move freely within the tube, container, etc., in response to shaking or other movement. The beads can be placed directly in contact with the sample to mechanically disrupt the microbial cells through movement of the beads through the sample. The beads may be of consistent or variable size, and may have any shape, such as smooth, rounded, geometric, irregular, etc. For example, the beads may vary in diameter from 0.1 mm to 3 mm, 0.1 mm to 2 mm, 0.1 mm to 1 mm, or 0.1 mm to 0.5 mm. The beads may be made of a variety of suitable hard materials, such as metal, plastic, garnet, carbide, carbide-tungsten, silica, glass, ceramic, etc. In certain examples, carbide beads may be used. The mechanical disruption may be carried out in a lysis buffer or solution, which may comprise various components, such as a buffer, chelator, salt(s), detergent(s), etc. According to some embodiments, the detergent may comprise cetyltrimethylammonium bromide (CTAB).

In further embodiments, a mechanically disrupted microbe sample is further exposed to an enzyme cocktail comprising multiple proteases or digestion enzymes, such as proteinase K, lysozyme, and mutanolysin, to produce a microbial lysate for use in gDNA extraction and purification. In some embodiments, the disrupted microbial sample or culture can be incubated with the enzyme cocktail at a specific temperature for a predetermined duration or length of time. In yet other embodiments, the microbial lysate may be further mixed with a binding buffer that may include a chaotropic agent and/or a chelating agent. In some embodiments, the microbial lysate may be filtered through a matrix or membrane that retains the microbial DNA. A wash buffer that may include an alcohol may be passed through the matrix or membrane to further purify the membrane or matrix retained or bound DNA. In some embodiments, the DNA may be eluted from the matrix or membrane with a saline buffer. However, other methods known in the art for DNA preparation and/or purification may be used to prepare, extract or purify gDNA from the microbial lysate.

The methods provided herein are particularly useful for efficiently extracting gDNA from a diverse sample of microbes. Existing methods may provide efficient gDNA extraction for some microbial species, but ineffective DNA extraction for other microbes having different cellular or extracellular structures that are or have been recalcitrant to extraction and purification or their genomic DNA. Surprisingly, the present inventors have succeeded in developing an improved DNA extraction method for purifying gDNA from a variety of microbial species and isolates. In one embodiment, the microbial sample or culture includes Gram-negative bacteria, Gram-positive bacteria, or a combination of both. In certain embodiments, the bacteria possess extensive peptidoglycan cell walls or other structural or biochemical features that make the microbe particularly resistant to lysis.

In some embodiments, effective gDNA extraction is accomplished through use of mechanical lysis methods followed by enzymatic lysis. In contrast, existing methods have typically used either mechanical disruption/lysis or enzymatic lysis, but not enzymatic lysis or digestion after or following mechanical lysis or disruption. Further, mechanical lysis and enzymatic lysis have not been used together because of the belief that mechanical lysis can damage the gDNA, and possibly the secondary and tertiary structures of the lytic enzyme(s), which would reduce their enzymatic activity. The present methods use mechanical disruption or lysis techniques followed by enzymatic digestion or lysis to produce unexpected and beneficial results - i.e., improved extraction of microbial gDNA.

Examples of mechanical disruption or lysis techniques which have been shown to be effective by the inventors include the use of "bead beating" techniques that utilize beads together with a shaker or shaking homogenizer. In particular embodiments, the use of garnet or carbide beads has been shown to be a particularly effective method of mechanical disruption. Commercial examples of shaking homogenizers or bead beaters include Powerlyzer^{™} (Mo Bio, Carlsbad, CA), Bio-Ruptor^{™} (Omni International, Kennesaw, GA), Mini Bead Beater^{™} (Cole-Parmer, Vernon Hills, IL), or Geno-Grinder^{™} (SpexSamplePrep, Metuchen, NJ). These instruments are used in conjunction with beads. The beads used for bead beating depend on the target tissue and the density of the tissues or cells to be disrupted or lysed. Harder tissues such as plant leaf tissue may require steel balls whereas softer tissues or cells may require a lighter matrix or bead such as glass, carbide, garnet or ceramic beads. Beads are available in different diameters, and methods of determining an optimal matrix and bead diameter are known in the art. For example, metal beads having an average diameter of approximately 1.5-3.0 mm, such as 2.38 mm, may be used with plant and seed samples. Ceramic beads with an average diameter of approximately 1.0 mm to 3.0 mm, such as 1.4 mm or 2.8 mm, may be used for human, animal, and plant tissue. Glass beads with an average diameter of less than 1 mm, for example 0.5 mm or 0.1 mm, may be used for microorganisms such as bacteria, fungi, yeast, and spores. Carbide beads with an average diameter of less than 0.5 mm, such as 0.25 mm, may be used for isolating DNA or RNA from microbes. Garnet beads with an average diameter of less than 1 mm, such as 0.15 mm or 0.70 mm, may be used to achieve rapid sample disruption.

Improved methods of mechanical disruption, together with methods involving mechanical disruption followed by enzymatic lysis, have led to unexpected and advantageous increases in gDNA extraction over divergent groups of microbes. The mechanical disruption may be carried out in a lysis buffer, which may include a detergent, a buffer, a denaturing agent, a reducing agent, a chelating agent, or any combination thereof. In yet other embodiments, the detergent is a cationic detergent such as, for example, cetyl trimethylammonium bromide (CTAB) or sodium dodecyl sulfate (SDS).

In certain embodiments, an enzyme cocktail for use in the present methods may include or comprise at least one proteinase, such as a serine protease, and at least one glycoside hydrolase. Examples of proteinases include serine proteases, such as proteases within the S8 family of the SB superfamily of serine proteases, for example proteinase K. Serine proteases further comprise any proteases that initiate the nucleophilic attack on the peptide (amide) bond through a serine residue at an active site. Examples of glycoside hydrolases include lysozyme, mutanolysin, and chitinase. In certain embodiments, the glycoside hydrolase is N-acetyl muramidase, N-acetyl muramide glycanhydrolase, muramidase, lysozyme, chitinase, chitosanase, or mutanolysin. Glycoside hydrolases may comprise any enzymes that catalyze the hydrolysis of glycosidic bonds in complex sugars (e.g. the peptidoglycan cell wall). According to some embodiments, the enzyme cocktail may comprise proteinase K, lysozyme, and mutanolysin. Such enzyme cocktail may further include one or more additional enzymes, such as achromopeptidase, RNase A, lysostephin, labiase, and/or lysyl-endopeptidase, or any combination thereof. The enzyme cocktail may also contain another type of glycoside hydrolase, such as one or more of carboxylate, glucosidase, N-acetylhexosaminidase, lactase, amylase, sucrase, maltase, neuraminidase, invertase, and/or hyaluronidase. The enzyme cocktail may further comprise various other components, such as a buffer, chelating agent, salts, etc.

In another embodiment, a binding buffer may be added to the microbial lysate following enzymatic digestion for use in purifying gDNA by binding to a matrix, substrate, membrane, column, etc., and subsequent elution. The binding buffer may include guanidine hydrochloride or another chaotropic agent. In yet another embodiment, the binding buffer may include ethylenediaminetetraacetic acid (EDTA) or other chelating agent.

In some embodiments, the mechanically disrupted microbial sample or culture may be incubated at a specific or predetermined temperature (or temperature range) with or without the enzyme cocktail, such as within a range from about 25°C to about 60°C, or about 25°C, about 37°C, about 45°C, or about 55°C. Incubation can proceed, for example, in a temperature-controlled incubator, oven, or water bath. In some embodiments, the mechanically disrupted microbial sample or culture may be incubated at a specific temperature (or temperature range) for a predetermined period of time, such as about 5, about 10, or about 15, about 20, about 25, about 30, about 35, about 40, about 45, about 50, about 55, or about 60 minutes. In some embodiments, the disrupted microbial sample or culture can be mixed, shaken, stirred, vortexed, sonicated, or otherwise disrupted at various time points after the mechanical disruption and before, during, or after the enzymatic incubation period.

In various embodiments, the lysed microbial samples or cultures are filtered through a membrane or substrate that retains the DNA. Examples of such substrates are known to those of skill in the art and may include, for instance, borosilicate glass or nylon. In certain embodiments, the DNA retained by the substrate is washed with a wash buffer that may include an alcohol, such as ethanol. In other embodiments, the DNA retained by the substrate may be washed multiple times, and subsequently eluted off the substrate with an elution buffer.

In some embodiments, microbial cultures, samples or isolates, which may comprise a large number of microbial cells, may be disrupted and lysed to successfully extract and isolate their DNA for subsequent analysis in a single assay. A microbial culture or sample may comprise a single or only a few microbial species(s) or isolate(s), or a variety of microbial species or strains. For example, a microbial sample may be taken or isolated from the environment and/or comprise a large number of different microbial species or strains. Alternatively, a microbial sample may be taken from a colony and generally comprise a single microbial species, strain or isolate. In another aspect, DNA is extracted from a microbial sample or isolates taken directly from soil or plants to help identify microbes native to a crop plant or soil environment. In particular embodiments, a large number of different crops, growing in a variety of soils, are harvested so that the associated microbes can be identified by analysis of the DNA extracted using the disclosed methods.

In other embodiments, the methods disclosed herein can extract and isolate DNA not only from non-spore-forming bacteria, but also, surprisingly, from spore-forming bacteria. In a non-limiting example, the methods disclosed herein can be used to extract and isolate DNA from Gram-negative bacteria which are generally resistant to existing lysis techniques.

In one embodiment, the present disclosure provides a method that can be scaled up to extract gDNA from multiple samples simultaneously, such as by using microtitre plates that have, for example, 96, 192, 384, 1536, or 3456 wells. In another embodiment, the method can extract gDNA from a mixed bacterial population including a broad range of common and recalcitrant microbial strains or isolates. In some embodiments, the mixed bacterial population represents the microbiota from environmental samples such as, for instance, plants or soil.

In certain embodiments, the extracted DNA includes polynucleotides having at least 10 kilobases (kb), at least 25 kb, at least 50 kb, at least 100 kb, at least 250 kb, at least 500 kb, or at least 1000 kb in length.

The use of a combination of at least one proteinase, such as a serine protease, and at least one glycoside hydrolase, N-acetylmuramidase, N-acetylmuramide glycanhydrolase, muramidase or muralytic enzyme, such as a lysozyme, mutanolysin, chitosanase, and/or chitinase, in a single lysis step and container avoids the use of multiple extraction steps or techniques to obtain DNA from different microbial species, after or following mechanical disruption of those microbial species. In some embodiments, a proteinase K and lysozyme, or a combination of proteinase K, lysozyme and mutanolysin, may be combined in the single lysis step and container. Thus, the present utility lies in part on the increases in efficiency over prior methods. A first step towards characterization of a microbial community in a sample can be the extraction of DNA from each of the various microbes in the sample. With existing methods, if particular microbes within the sample are resistant to DNA extraction, then their DNA, and thus their very presence, may be underrepresented or absent from the purified DNA preparation and subsequent analysis. Rather than treat each microbial culture with multiple DNA extraction methodologies which may each be successful at lysing a subset of microbes, the methods disclosed herein offer a rapid, streamlined process for extracting DNA from multiple genera and species of microorganisms including many recalcitrant microbes in a single reaction and/or container. Thus, a significant amount of time and labor can be saved by performing gDNA extraction simultaneously from microbial species present in the same container or sample.

In some embodiments, the methods described herein release or extract DNA from cells by lysing the cells with a cocktail of enzymes. The resulting DNA is particularly suited for further analysis by a variety of techniques that require high-quality, intact DNA.

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art. Unless otherwise noted, terms are to be understood according to conventional usage by those of ordinary skill in the relevant art.

Diverse plant-associated microorganisms can positively impact plant health and physiology in a variety of ways. The term "beneficial microbes," as used herein, encompasses a wide range of improved plant properties, including, for example without limitation, improved nitrogen fixation, improved root development, increased leaf area, increased plant yield, increased seed germination, increased photosynthesis, or an increased in accumulated biomass of the plant. Thus, as non-limiting examples, the microbes may produce an increase in nitrogen fixation, or an above stated increase in total root weight, or in leaf area or in plant product yield (e.g., an increase in plant product weight), or an increased percentage of seeds that germinate within 10 days or 14 days or 30 days, or rate of photosynthesis (e.g., determined by CO₂ consumption) or accumulated biomass of the plant (e.g., determined by weight of the plant). The yield can be determined using any suitable method, for example, bushels or pounds of plant product (e.g., seeds, kernels, etc.) per acre of planting.

However, identification of beneficial microorganisms can depend, in large part, on DNA analyses. If beneficial microbes are resistant to DNA lysis and extraction, then their DNA will be absent or below the level of detection necessary to identify their presence in the sample. Thus, the identification of these strains as possible inoculants in seed treatment compositions might be dependent on their susceptibility to DNA extraction methods for subsequent DNA analyses. However, present methods are provided to overcome these limitations through efficient extraction and isolation of DNA from microorganisms that have been resistant to lysis and DNA extraction.

Effective taxonomic identification can also be dependent on effective methods for DNA extraction where the taxonomy is defined by a molecular assay, marker or sequencing. Microorganisms can often be distinguished based on direct microscopic analysis (do all of the cells in a sample look the same on examination), staining characteristics, simple molecular analysis (such as a simply restriction fragment length polymorphism (RFLP) determination), and so forth. Taxonomic identification of a microorganism can involve up to several different levels of analysis, and each analysis can be based on a different characteristic of the organism. Such taxonomic analyses can include nucleic acid-based analysis (e.g., analysis of individual specific genes, either as to their presence or their exact sequence, or expression of a particular gene or a family of genes), protein-based analysis (e.g., at a functional level using direct or indirect enzyme assays, or at a structural level using immuno-detection techniques), etc.

One of ordinary skill in the art will appreciate that a wide variety of nucleic acid-based techniques are known and can be useful in obtaining the taxonomic identification of a given microorganism. These techniques can be used to identify cells by gene sequence or to identify cells that have particular genes or gene families. Common gene families, genes or other sequences useful for taxonomic studies include the 16S gene family, actin gene family, recombinase A (*recA*) gene family, and the internal transcribed spacer (ITS) sequence. These methods typically include amplifying and sequencing genes or sequences from very small numbers of cells, and therefore often overcome the problems of concentrating cells or samples and their DNA from more dilute suspensions.

The term "nucleic acid amplification" or "DNA amplification" generally refers to techniques that increase the number of copies of a nucleic acid molecule in a sample or specimen. Techniques useful for nucleic acid amplification are well known in the art. An example of nucleic acid amplification is the polymerase chain reaction (PCR), in which a biological sample collected from a subject is contacted with a pair of oligonucleotide primers, under conditions that allow for the hybridization of the primers to nucleic acid template in the sample. The primers are extended under suitable conditions, dissociated from the template, and then re-annealed, extended, and dissociated to amplify the number of copies of the nucleic acid. Other examples of *in vitro* amplification techniques include strand displacement amplification; transcription-free isothermal amplification; repair chain reaction amplification; ligase chain reaction; gap filling ligase chain reaction amplification; coupled ligase detection and PCR; and RNA transcription-free amplification. Primers have also been designed routinely, and new ones are continually being designed, for individual species or phylogenetic groups of microorganisms. Such narrowly targeted primers can be used with the methods described herein to screen and/or identify specifically only the microorganisms of interest.

Methods for preparing and using nucleic acid primers are described, for example, in Sambrook et al. (In Molecular Cloning: A Laboratory Manual, CSHL, New York, 1989), Ausubel et al. (ed.) (In Current Protocols in Molecular Biology, John Wiley & Sons, New York, 1998). Amplification primer pairs can be derived from a known sequence, for example, by using computer programs intended for that purpose such as Primer (Whitehead Institute for Biomedical Research, Cambridge, Mass.). One of ordinary skill in the art will appreciate that the specificity of a particular probe or primer increases with its length. Thus, for example, a primer comprising 30 consecutive nucleotides of an rRNA-encoding nucleotide or flanking region thereof will anneal to a target sequence with a higher specificity than a corresponding primer of only 15 nucleotides. Thus, in order to obtain greater specificity, probes and primers can be selected that comprise at least 20, 25, 30, 35, 40, 45, 50 or more consecutive nucleotides of a target nucleotide sequence such as the 16S rRNA.

Common techniques for the preparation of nucleic acids useful for nucleic acid applications (e.g., PCR) include phenol/chloroform extraction or use of one of the many DNA extraction kits that are available on the market. Another way that DNA can be amplified is by adding cells directly to the nucleic acid amplification reaction mix and relying on the denaturation step of the amplification to lyse the cells and release the DNA.

The product of nucleic acid amplification reactions may be further characterized by one or more of the standard techniques that are well known in the art, including electrophoresis, restriction endonuclease cleavage patterns, oligonucleotide hybridization or ligation, and/or nucleic acid sequencing. When in hybridization techniques are used for cell identification purposes, a variety of probe labeling methods can be useful, including fluorescent labeling, radioactive labeling and non-radioactive labeling. When nucleic acid sequencing techniques are used, homology search for the nucleotide sequence of the amplified nucleic acid molecules can be conducted using various databases of known sequences, including but not limited to DDBJ/GenBank/EMBL databases.

Samples or cultures of the microorganisms from which DNA is extracted using the methods herein can be prepared using standard microbial culture techniques known in the art. Cultures of one or more microorganisms may be taken from a source (e.g., plant, soil, leaf, surface, etc.) and grown and/or selected in a solid, semi-solid or liquid media as known in the art. Culturing or growth can be carried out under aerobic or anaerobic conditions with a variety of different nutrients, salts, buffers, etc., and under various growth conditions. Growth can also be effected in a bioreactor for larger scale production.

A bioreactor refers to a device or system that supports a biologically active environment. As described herein, a bioreactor is a vessel in which microorganisms can be grown. A bioreactor may be any appropriate shape or size for growing the microorganisms. A bioreactor may range in size and scale from 10 mL to many liters or cubic meters and may be made of stainless steel or any other appropriate material as known and used in the art. The bioreactor may be a batch type bioreactor, a fed batch type or a continuous-type bioreactor (e.g., a continuous stirred reactor). For example, a bioreactor may be a chemostat as known and used in the art of microbiology for growing and harvesting bacteria. A bioreactor may be obtained from any commercial supplier (see also Bioreactor System Design, Asenjo & Merchuk, CRC Press, 1995).

For small scale operations, a batch bioreactor may be used, for example, to test and develop new processes, and for processes that cannot be converted to continuous operations.

Microorganisms grown in a bioreactor may be suspended or immobilized. Growth in the bioreactor is generally under aerobic conditions at suitable temperatures and pH for growth as provided herein. For example, cell growth may be achieved at temperatures between 5 and 37°C, such as in the range of 15 to 30°C, 15 to 28°C, 20 to 30°C, or 15 to 25°C. The pH of the nutrient medium may vary between 4.0 and 9.0, such as in the range of pH 4.0 to 7.0, or 4.5 to 6.5, or pH 5.0 to 6.0. As an example, microorganisms may be grown for 20-72 hours after inoculation.

Optimal conditions for growth and cultivation of microorganisms will likely depend on the particular strain(s) or isolate(s) or population of strains or isolates. However, a person skilled in the art will often be able to determine the appropriate growth and culturing conditions including any essential nutrients and conditions based on proven culturing methods for other microorganisms and/or through routine experimentation. Microbes may be grown in aerobic liquid cultures on media which contain sources of carbon, nitrogen, and inorganic salts that can be assimilated by the microorganism and supportive of efficient cell growth. Preferred carbon sources are hexoses such as glucose, but other sources that are readily assimilated such as amino acids, may be substituted. Many inorganic and proteinaceous materials may be used as nitrogen sources in the growth process. Preferred nitrogen sources are amino acids and urea but others include gaseous ammonia, inorganic salts of nitrate and ammonium, vitamins, purines, pyrimidines, yeast extract, beef extract, proteose peptone, soybean meal, hydrolysates of casein, distiller's solubles, and the like. Among the inorganic minerals that can be incorporated into the nutrient medium are the customary salts capable of yielding calcium, zinc, iron, manganese, magnesium, copper, cobalt, potassium, sodium, molybdate, phosphate, sulfate, chloride, borate, and like ions. Without being limited thereto, use of potato dextrose liquid medium for fungal antagonists and R2A broth premix for bacterial strains is preferred.

The skilled artisan will recognize that the methods provided herein can in principle be applied to extract DNA from a wide taxonomic variety of microorganisms including bacteria and fungi that are generally recalcitrant or resistant to other methods of lysis or DNA extraction. Present methods are not intended to be limited to particular cultures, species or cell types. For example, microbial cells that undergo complex forms of differentiation, filamentation, sporulation, etc., can also be used in methods provided herein.

In principle, the methods provided herein can be deployed or used for many different bacterial, fungal and other microbial genera and species. The methods described herein may be used with microbes that are important, useful or relevant to agriculture, horticulture, biomass production, and forestry. The methods described herein may also be used with microbes that are important, useful or relevant to medicine, veterinary uses, livestock, and the environment.

Thus, present methods have use with a broad range of bacteria including both Gram-negative and Gram-positive bacteria such as Actinobacteridae, Coriobacteridae Arthrobacter, Bacillus, Streptomyces, etc., and a broad range of fungi and other microorganisms.

Having described the invention in detail, it will be apparent that modifications, variations, and equivalent embodiments are possible without departing the scope of the invention defined in the appended claims. Furthermore, it should be appreciated that all examples in the present disclosure are provided as non-limiting examples.

### EXAMPLES

### Example 1

### Preparation of Bead Plates and Reagents

### Bead Plates

A full skirted Eppendorf twin.tec PCR plate rack (Fischer Scientific^{®}) was prepared with foil seal on the bottom with 0.25mm carbide beads (MoBio). A 2.2mL deepwell storage plate was inverted over the PCR plate rack, lining up the wells. The plates were quickly inverted while being held together, transferring the beads to the deepwell plate. The plate was sealed using PlateLoc with a foil seal. It was found to be advantageous to cover plates with a heat activated seal instead of an adhesive seal to prevent adhesion of the beads to the adhesive seal of the plate. Plates were stored at room temperature until needed.

### Lysis Buffer:

100 mM Tris HCl, pH 8
20 mM EDTA, pH 8
1.4 M NaCl
1.4 M guanidine HCl (GuHCl; CalBiochem)
2% cetyltrimethylammonium bromide (CTAB; CalBiochem)

### Enzyme Cocktail:

Enzyme cocktail was prepared as follows. 140uL was aliquoted into 8-strip PCR tubes and stored at -20C until ready to use. Each 8-strip tube contains enzyme cocktail for a 96 well plate.

| **Enzyme** | **10 plates** | **20 plates** | **30 plates** |
|---|---|---|---|
| Proteinase K, 100mg/mL (Amresco^{®}) | 1.2 mL | 2.4 mL | 3.6 mL |
| RNase A, 50mg/ml (Amresco^{®}) | 1.2 mL | 2.4 mL | 3.6 mL |
| Mutanolysin, 25KU/mL (Sigma Aldrich^{®}) | 1.5 mL | 3.0 mL | 4.5 mL |
| Lysozyme, 100mg/mL (Thermo Scientific^{®}) | 6.0 mL | 12.0 mL | 18.0 mL |
| Sterile ddH₂O | 0.1 mL | 0.2 mL | 0.3 mL |

### Pre-Binding Buffer:

6M GuHCl
20mM EDTA, pH 8
lOmM Tris HCl, pH 8
4% Triton X-100
Adjust to pH 4

### Binding Buffer:

83.3% Pre-Binding Buffer
10% Ethanol
7.3% Sterile ddH₂O

### Wash Buffer 1 (WB1):

50% Pre-Binding Buffer
50% Ethanol

### Wash Buffer 2 (WB2):

60% Ethanol
50 mM NaCl
10 mM Tris HCl, pH 8
0.5 mM EDTA, pH 8

### T₁₀E_{0.1}:

lOmM Tris HCl, pH 7
EDTA, pH 8.0

### T₁₀E₁:

lOmM Tris HCl, pH 7
1mM EDTA, pH 8.0

### Example 2

### Microbial Genomic DNA Extraction

A large diversity of both Gram-positive and Gram-negative bacteria were cultured in R2B liquid media in 50 mL conical tubes or 2.2 mL storage plates for 2-4 days in a 29°C shaker. 1.5 mL of each culture was added to the wells of a 2.2mL storage plate and centrifuged at 4500RPM for 45 minutes in a 4°C centrifuge. The supernatant was discarded, pellets were resuspended in 300µL lysis buffer and mixed using a pipet. The entire volume was transferred to a prepared bead plate with 0.25 mm carbide beads as described in Example 1. The plate was sealed using a PlateLoc with an aluminum seal and placed on a plate shaker (Retsch Mill Mixer - MM400) and run for 10 minutes at 20/s. The plate was centrifuged for 6 minutes at 4500RPM. Supernatant was transferred to a new deepwell plate, and 11uL enzyme cocktail was added to each well. The plate was sealed with PlateLoc and vortexed for 10-15 seconds (a total of 3 times). 800µL of binding buffer was added to each well and mixed via pipette (total volume ~1.1 mL per well).

700uL of lysate was transferred to a Pall Acroprep filter plate (VWR^{®}) stacked on a clean 2.2mL deepwell storage plate. Plates were centrifuged for 3 minutes at 4500RPM. The remaining volume was transferred to the wells of the filter plate and centrifuged for 3 minutes at 4500RPM. 500uL WB1 was added to each well of the filter plate, and the plate was centrifuged for 3 minutes at 4500 RPM. Flow through was discarded and the filter plate was placed back on the storage plate. 700uL of WB2 was added to each well, and the plate was centrifuged for 3 minutes at 4500RPM. An additional 700uL of WB2 was added to each well, and the plate was centrifuged for 5 minutes at 4500RPM. 100 µL T₁₀E_{0.1} at 37-45°C was added to the center of each membrane, and the filter plate was transferred to an Eppendorf twin.tec PCR plate. The plate was centrifuged for 3 minutes at 4500RPM, sealed using PlateLoc, and stored at -20°C.

Microbial DNA was eluted from each well with 100 µL of TE buffer (10 mM Tris HCl, pH 7, and 0.1 mM EDTA, pH 8). Isolated gDNA was quantified using Life Technologies^{®} (Grand Island, NY, USA) Quant-iT^{™} PicoGreen^{®} dsDNA Reagent.

### Example 3

### Microbial Genomic DNA Extraction

To test the efficiency of the gDNA extraction method of Example 2, gDNA was extracted from a plate of Actinomyces (known to present difficulty in extracting gDNA), and compared with a commercial gDNA extraction kit, Kit 1.

As shown in Table 1, the extraction method described herein in Example 2 ("AG") produced increased gDNA concentrations compared to Kit 1 for 48 of 94 total samples in the plate. The average gDNA concentration increase was 91% when using the AG method over Kit 1. In addition, the AG method returned a larger number of isolates with concentrations greater than 20 ng/µL, the minimal concentration for a number of applicable downstream applications (25 isolates with AG method, compared to 19 isolates with Kit 1).

**Table 1. Comparison of disclosed methods for gDNA extraction with commercial kit.**

| **Wells** | **Kit 1 (ng/pL)** | **Kit 1 (Total ng)** | **AG (ng/pL)** | **AG (Total ng)** | **% difference AG v. Kit 1** |
|---|---|---|---|---|---|
| A1 | 13.796 | 1034.7 | 19.729 | 1479.7 | 43.0052 |
| B1 | 0.249 | 18.7 | 0.587 | 44.0 | 135.7430 |
| C1 | 0.394 | 29.6 | 0.463 | 34.7 | 17.5127 |
| D1 | 1.752 | 131.4 | 2.824 | 211.8 | 61.1872 |
| E1 | 0.862 | 64.7 | 0.509 | 38.2 | -40.9513 |
| F1 | 13.742 | 1030.7 | 22.482 | 1686.2 | 63.6006 |
| G1 | 1.162 | 87.2 | 0.534 | 40.1 | -54.0448 |
| H1 | 3.521 | 264.1 | 12.151 | 911.3 | 245.1008 |
| A2 | 1.762 | 132.2 | 2.255 | 169.1 | 27.9796 |
| B2 | 0.261 | 19.6 | 0.322 | 24.2 | 23.3716 |
| C2 | 11.195 | 839.6 | 10.577 | 793.3 | -5.5203 |
| D2 | 0.258 | 19.4 | 8.391 | 629.3 | 3152.3256 |
| E2 | 5.986 | 449.0 | 9.139 | 685.4 | 52.6729 |
| F2 | 0.988 | 74.1 | 0.553 | 41.5 | -44.0283 |
| G2 | 5.452 | 408.9 | 3.867 | 290.0 | -29.0719 |
| H2 | 1.179 | 88.4 | 2.556 | 191.7 | 116.7939 |
| A3 | 2.044 | 153.3 | 6.708 | 503.1 | 228.1800 |
| B3 | 0.275 | 20.6 | 1.942 | 145.7 | 606.1818 |
| C3 | 3.5 | 262.5 | 10.334 | 775.1 | 195.2571 |
| D3 | 0.323 | 24.2 | 0.443 | 33.2 | 37.1517 |
| E3 | 8.465 | 634.9 | 10.945 | 820.9 | 29.2971 |
| F3 | 34.139 | 2560.4 | 14.378 | 1078.4 | -57.8839 |
| G3 | 3.665 | 274.9 | 1.081 | 81.1 | -70.5048 |
| H3 | 7.893 | 592.0 | 9.809 | 735.7 | 24.2747 |
| A4 | 4.324 | 324.3 | 13.61 | 1020.8 | 214.7549 |
| B4 | 0.433 | 32.5 | 3.227 | 242.0 | 645.2656 |
| C4 | 10.378 | 778.4 | 28.289 | 2121.7 | 172.5862 |
| D4 | 14.156 | 1061.7 | 33.478 | 2510.9 | 136.4934 |
| E4 | 12.614 | 946.1 | 6.524 | 489.3 | -48.2797 |
| F4 | 29.231 | 2192.3 | 35.079 | 2630.9 | 20.0062 |
| G4 | 0.304 | 22.8 | 0.313 | 23.5 | 2.9605 |
| H4 | 0.401 | 30.1 | 0.386 | 29.0 | -3.7406 |
| A5 | 42.87 | 3215.3 | 135.861 | 10189.6 | 216.9139 |
| B5 | 3.546 | 266.0 | 40.035 | 3002.6 | 1029.0186 |
| C5 | 0.545 | 40.9 | 0.383 | 28.7 | -29.7248 |
| D5 | 2.55 | 191.3 | 0.811 | 60.8 | -68.1961 |
| E5 | 23.113 | 1733.5 | 79.394 | 5954.6 | 243.5037 |
| F5 | 32.682 | 2451.2 | 103.593 | 7769.5 | 216.9726 |
| G5 | 5.979 | 448.4 | 0.889 | 66.7 | -85.1313 |
| H5 | 45.507 | 3413.0 | 99.587 | 7469.0 | 118.8389 |
| A6 | 0.38 | 28.5 | 0.256 | 19.2 | -32.6316 |
| B6 | 2.577 | 193.3 | 4.365 | 327.4 | 69.3830 |
| C6 | 23.13 | 1734.8 | 48.384 | 3628.8 | 109.1829 |
| D6 | 23.935 | 1795.1 | 48.856 | 3664.2 | 104.1195 |
| E6 | 139.004 | 10425.3 | 115.264 | 8644.8 | -17.0786 |
| F6 | 105.779 | 7933.4 | 66.729 | 5004.7 | -36.9166 |
| G6 | 0.965 | 72.4 | 0.441 | 33.1 | -54.3005 |
| H6 | 32.669 | 2450.2 | 51.078 | 3830.9 | 56.3501 |
| A7 | 7.285 | 546.4 | 8.682 | 651.2 | 19.1764 |
| B7 | 0.354 | 26.6 | 0.255 | 19.1 | -27.9661 |
| C7 | 14.262 | 1069.7 | 44.106 | 3308.0 | 209.2554 |
| D7 | 1.745 | 130.9 | 0.254 | 19.1 | -85.4441 |
| E7 | 3.841 | 288.1 | 1.251 | 93.8 | -67.4304 |
| F7 | 0.936 | 70.2 | 0.353 | 26.5 | -62.2863 |
| G7 | 15.66 | 1174.5 | 7.866 | 590.0 | -49.7701 |
| H7 | 14.818 | 1111.4 | 11.368 | 852.6 | -23.2825 |
| A8 | 0.479 | 35.9 | 0.26 | 19.5 | -45.7203 |
| B8 | 0.533 | 40.0 | 0.662 | 49.7 | 24.2026 |
| C8 | 1.489 | 111.7 | 0.36 | 27.0 | -75.8227 |
| D8 | 1.346 | 101.0 | 0.262 | 19.7 | -80.5349 |
| E8 | 19.46 | 1459.5 | 12.939 | 970.4 | -33.5098 |
| F8 | 1.162 | 87.2 | 0.256 | 19.2 | -77.9690 |
| G8 | 10.684 | 801.3 | 2.464 | 184.8 | -76.9375 |
| H8 | 8.769 | 657.7 | 4.276 | 320.7 | -51.2373 |
| A9 | 2.201 | 165.1 | 1.713 | 128.5 | -22.1717 |
| B9 | 1.537 | 115.3 | 2.503 | 187.7 | 62.8497 |
| C9 | 6.669 | 500.2 | 2.66 | 199.5 | -60.1140 |
| D9 | 9.886 | 741.5 | 9.867 | 740.0 | -0.1922 |
| E9 | 4.32 | 324.0 | 2.633 | 197.5 | -39.0509 |
| F9 | 12.206 | 915.5 | 5.896 | 442.2 | -51.6959 |
| G9 | 13.605 | 1020.4 | 4.276 | 320.7 | -68.5704 |
| H9 | 12.431 | 932.3 | 1.732 | 129.9 | -86.0671 |
| A10 | 11.249 | 843.7 | 30.321 | 2274.1 | 169.5440 |
| B10 | 9.044 | 678.3 | 5.835 | 437.6 | -35.4821 |
| C10 | 12.985 | 973.9 | 11.69 | 876.8 | -9.9730 |
| D10 | 7.132 | 534.9 | 10.055 | 754.1 | 40.9843 |
| E10 | 10.058 | 754.4 | 13.17 | 987.8 | 30.9405 |
| F10 | 9.701 | 727.6 | 3.899 | 292.4 | -59.8083 |
| G10 | 14.215 | 1066.1 | 9.7 | 727.5 | -31.7622 |
| H10 | 21.403 | 1605.2 | 13.718 | 1028.9 | -35.9062 |
| A11 | 11.375 | 853.1 | 41.208 | 3090.6 | 262.2681 |
| B11 | 1.133 | 85.0 | 3.454 | 259.1 | 204.8544 |
| C11 | 2.59 | 194.3 | 1.491 | 111.8 | -42.4324 |
| D11 | 3.881 | 291.1 | 11.236 | 842.7 | 189.5130 |
| E11 | 27.925 | 2094.4 | 65.208 | 4890.6 | 133.5112 |
| F11 | 42.803 | 3210.2 | 21.878 | 1640.9 | -48.8868 |
| G11 | 39.719 | 2978.9 | 110.122 | 8259.2 | 177.2527 |
| H11 | 90.097 | 6757.3 | 80.129 | 6009.7 | -11.0636 |
| A12 | 45.727 | 3429.5 | 53.773 | 4033.0 | 17.5957 |
| B12 | 38.75 | 2906.3 | 61.304 | 4597.8 | 58.2039 |
| C12 | 18.656 | 1399.2 | 30.605 | 2295.4 | 64.0491 |
| D12 | 4.023 | 301.7 | 30.2 | 2265.0 | 650.6836 |
| E12 | 20.407 | 1530.5 | 15.068 | 1130.1 | -26.1626 |
| F12 | 2.349 | 176.2 | 0.34 | 25.5 | -85.5258 |
| G12 | 0.246 | 18.5 | 0.245 | 18.4 | empty control well |
| H12 | 0.25 | 18.8 | 0.241 | 18.1 | empty control well |

### Example 4

### Cationic Detergent Evaluation

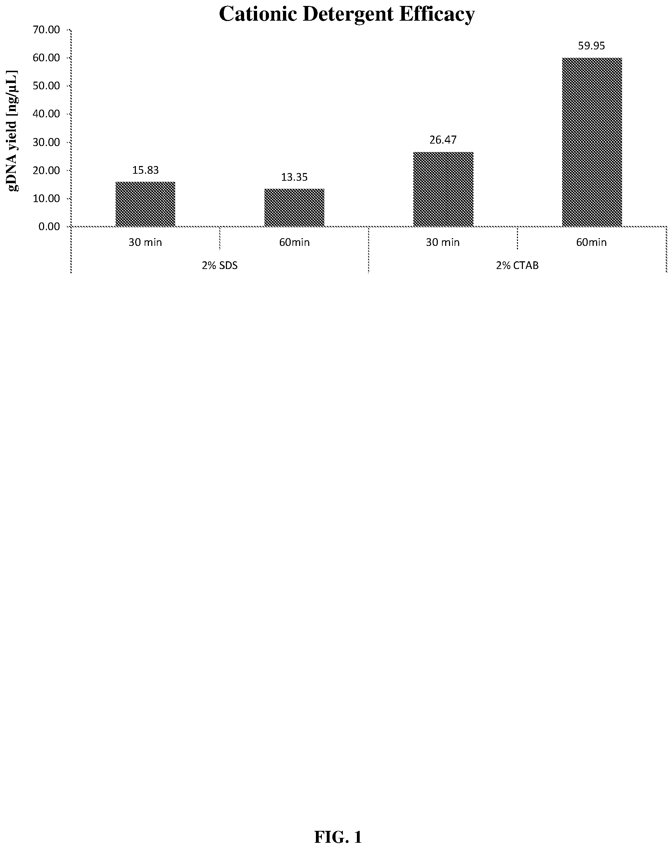

Microbial gDNA was extracted according to the methods provided herein and compared to the same method using 2% SDS (sodium dodecyl sulfate) instead of 2% CTAB (cetyltrimethylammonium bromide). Extraction using CTAB increased the recovered gDNA concentration by 78% when the lysis reaction was incubated for 1 hour. See Figure 1.

### Example 5

### Lysozyme Evaluation

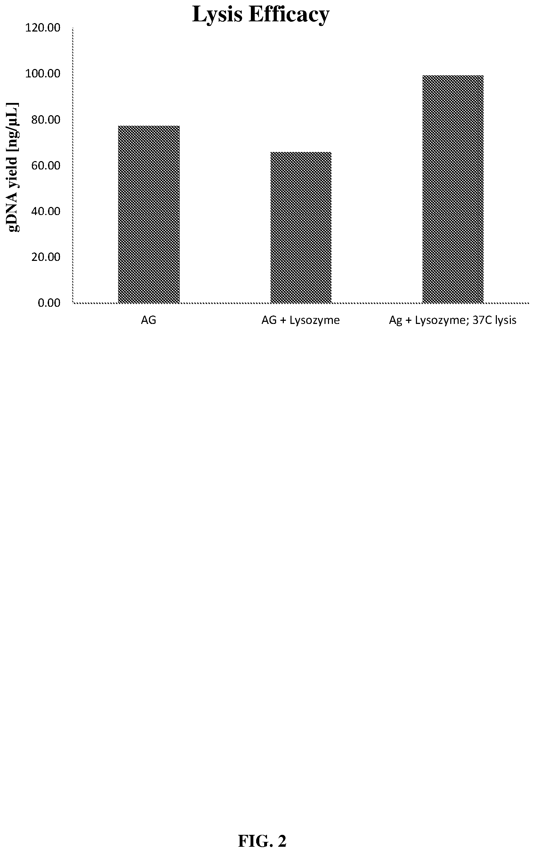

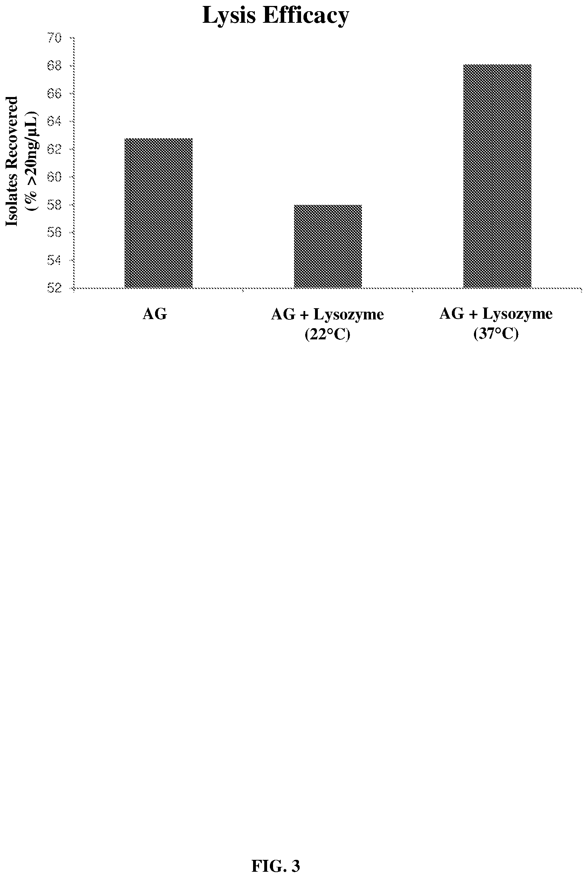

Microbial gDNA was extracted according to methods described herein with and without lysozyme in the lysis buffer. Standard lysis conditions for this experiment included lysis buffer with 0.4 mg/mL proteinase K and 125 U/mL mutanolysin. In certain samples, lysozyme was added to the standard lysis buffer at 0.1 mg/mL. Results indicated that the addition of lysozyme to lysis buffer already including proteinase K and mutanolysin increased both the average yield (i.e., concentration) of extracted gDNA, as well as total number of isolates recovered. See Figures 2 and 3, respectively.

### Example 6

### Incubation Time Titration

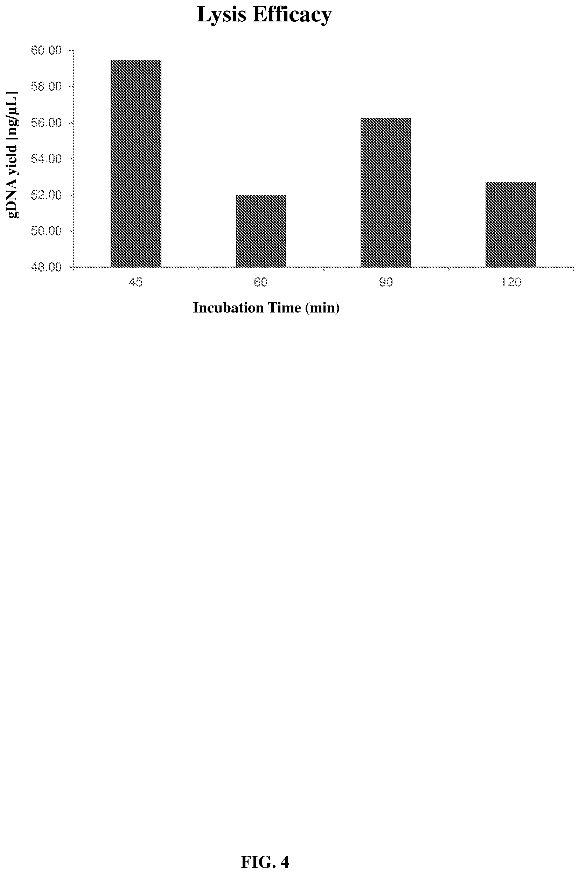

Microbial gDNA was extracted as described herein using varying periods of incubation on a microbial mixture of 4 Gram-positive and 8 Gram-negative strains. Results indicated that incubation with a lysis buffer comprising proteinase K, mutanolysin, and lysozyme resulted in the highest yield of gDNA following a 45 minute incubation period at 37°C. See Figure 4.

### Example 7

### Lysozyme Concentration Titration

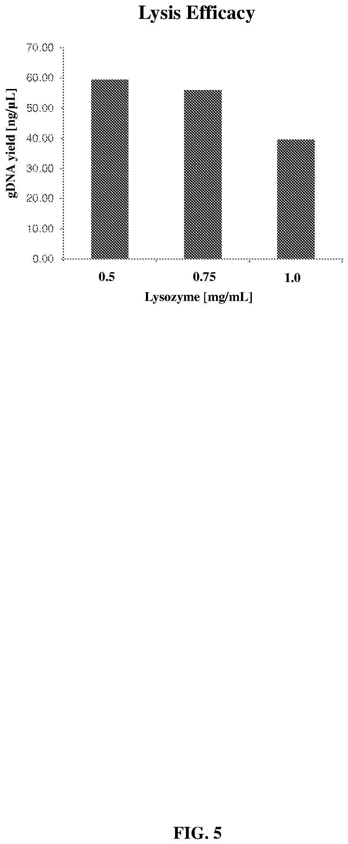

Microbial gDNA was extracted as described herein using varying concentrations of lysozyme in the lysis buffer containing proteinase K and mutanolysin. Results indicated that incubation with 0.5 mg/mL lysozyme resulted in the highest yield of gDNA. See Figure 5.

### Example 8

### Multi-Sample Validation

Microbial gDNA was extracted as described herein (AG method) and compared to gDNA prepared using various commercial DNA extraction methods (Kit 1, Kit 2, and Kit 3). Microbial cultures from 376 isolates, representing 65 genera (13 Gram-positive and 52 Gram-negative) were used as starting material and grown using micro-well plates covered with a gas-permeable foil seal, in liquid media, overnight at 30°C on a plate shaker.

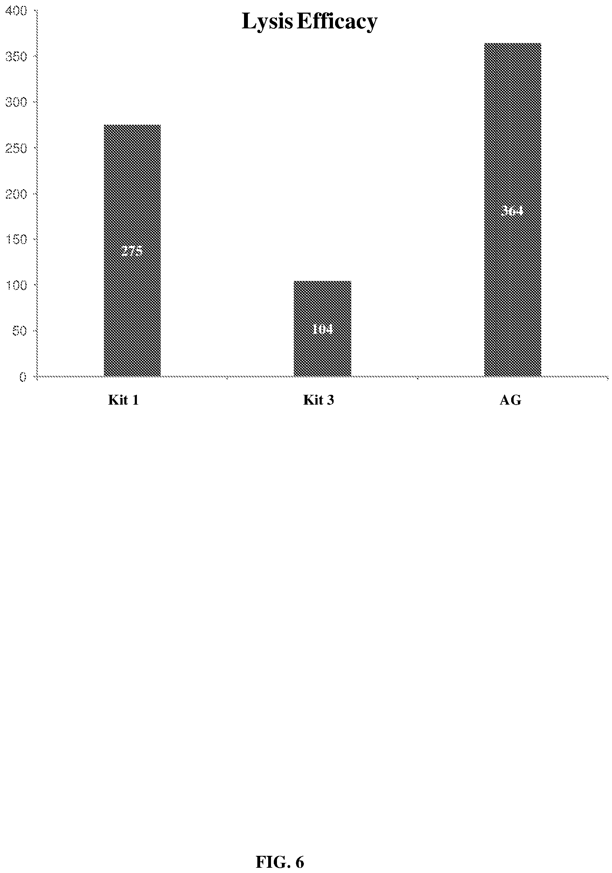

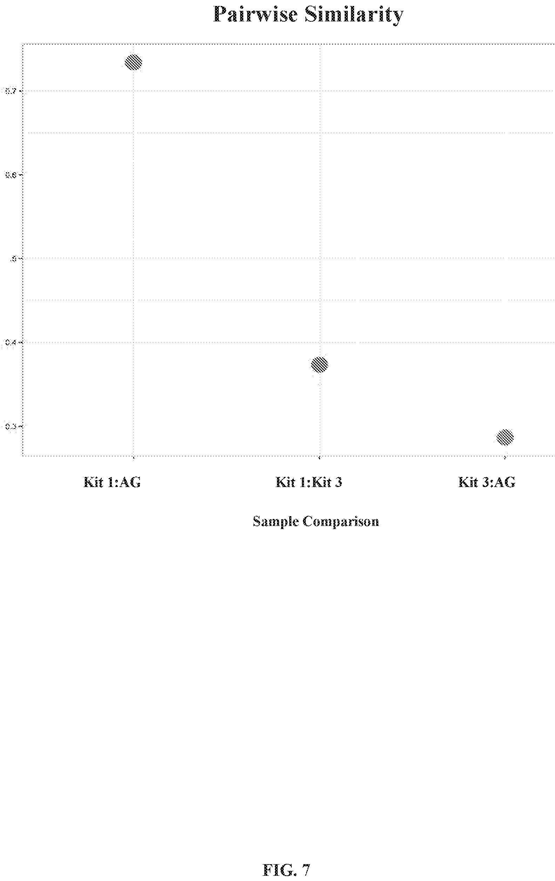

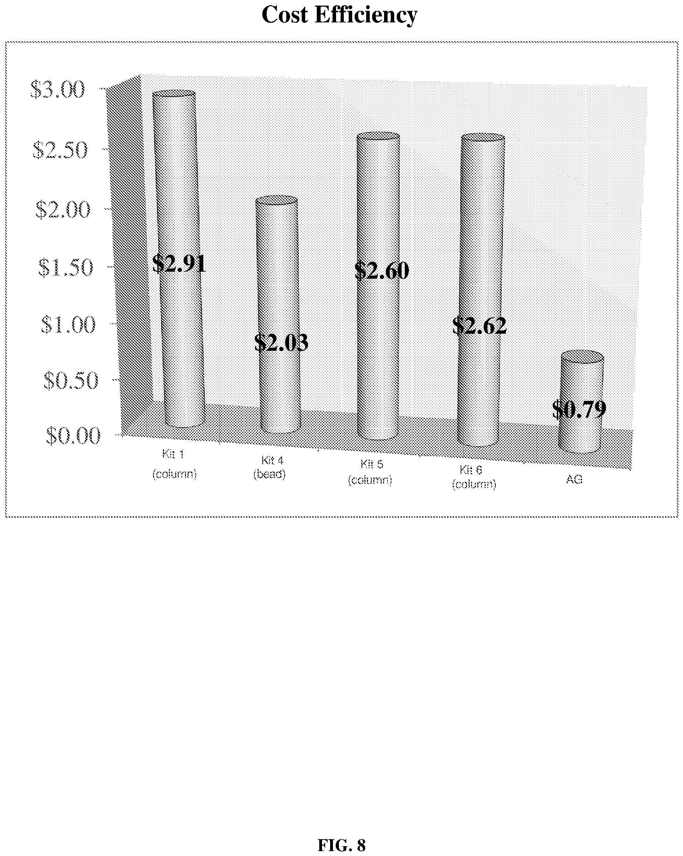

Kit performance was evaluated based on the ability to obtain a gDNA threshold concentration of 20 ng/µl. Results indicate that using the method described herein, gDNA above the threshold concentration was obtained for 364 isolates while the Kit 1 and Kit 3 methods returned only 275 and 104 isolates, respectively. See Figure 6. Pairwise similarities between the taxonomic profiles of isolated successfully extracted using the different methodologies were calculated using the binary Jaccard index. Results indicate that the taxonomic profiles of the isolates successfully extracted using each method were significantly different (Kruskal-Wallis ANOVA, p<1e⁻¹⁶). See Figure 7. In addition, results indicate that the method described herein yields more gDNA across a greater diversity of microbial genera compared with other commercially available kits (Kit 1, Kit 4, Kit 5, and Kit 6) at a reduced cost. See Figure 8.

### Example 9

### Environmental Isolates

Microbial gDNA can be extracted as described herein on a wide range of environmental isolates. For example, the method described herein can be useful in identifying previously unknown isolates of microbes native to the soil(s) used for cultivating various agricultural crop plants or from other environments, and/or improving gDNA extraction from isolates that may be recalcitrant to such gDNA extraction using conventional techniques.

### Example 10

### Homogenization & Enzymatic Mechanisms

Microbial gDNA can be extracted as described in Example 2 using alternate methods of mechanical homogenization prior to enzymatic lysis. For instance, various types of beads or particles other than carbide beads can also be used to mechanically disrupt microbial cell walls prior to incubation with proteinase K, mutanolysin, and lysozyme. Moreover, additional enzymes can be used to further expand the lytic capabilities of the instant method.

## Claims

1. A method for extracting genomic DNA (gDNA) from a microbial sample comprising the steps of:
a) subjecting the microbial sample to mechanical disruption;
b) subsequent to step (a) contacting the mechanically disrupted microbial sample with an enzyme cocktail comprising a proteinase and a glycoside hydrolase to produce a lysate of the microbial sample; and
c) purifying microbial gDNA from the lysate of the microbial sample.

2. The method of claim 1, wherein mechanical disruption comprises the use of beads.

3. The method of claim 2, wherein the beads are
(a) garnet beads, carbide beads, metal beads, glass beads, or ceramic beads;
(b) have a diameter between 0.1 mm and 3.0 mm; or
(c) have a diameter between 0.1 mm and 0.5 mm.

4. The method of claim 1, wherein mechanical disruption comprises the use of a shaking homogenizer.

5. The method of claim 1, wherein the microbial sample subjected to mechanical disruption is in a lysis buffer, and wherein the lysis buffer comprises a cationic detergent.

6. The method of claim 1, wherein the mechanically disrupted microbial sample is contacted with the enzyme cocktail for about 30 to 60 minutes or between about 25°C and about 60°C.

7. The method of claim 1, wherein the proteinase is a serine protease.

8. The method of claim 1, wherein the proteinase is at a concentration of between about 0.1 and about 1.0 mg/mL.

9. The method of claim 1, wherein the glycoside hydrolase is a N-acetyl muramidase, N-acetyl muramide glycanhydrolase, or muramidase.

10. The method of claim 1, wherein the enzyme cocktail further comprises a second glycoside hydrolase, wherein the glycoside hydrolase is different than the second glycoside hydrolase.

11. The method of claim 1, wherein the enzyme cocktail comprises additional enzymes selected from the group consisting of RNase A, lysostephin, labiase, lysyl- endopeptidase, and a combination thereof.

12. The method of claim 1, wherein purifying microbial gDNA from the lysate comprises:
1) adding a binding buffer comprising a chaotropic agent and a chelating agent to the lysate of the microbial sample to produce a binding mixture;
2) filtering the binding mixture through a DNA-binding matrix;
3) filtering a wash buffer, comprising an alcohol, through the matrix; and
4) filtering an elution buffer through the matrix to produce an eluate, wherein the eluate comprises the microbial gDNA.

13. The method of claim 1, wherein the microbial sample comprises Gram-negative bacteria, Gram-positive bacteria, or a combination of both.

14. The method of claim 1, wherein the microbial sample comprises environmental isolates.

## Patentansprüche

1. Verfahren zur Extraktion von genomischer DNA (gDNA) aus einer mikrobiellen Probe, umfassend die folgenden Schritte:
a) Durchführen eines mechanischen Aufschlusses mit der mikrobiellen Probe;
b) im Anschluss an Schritt (a) Inkontaktbringen der mechanisch aufgeschlossenen mikrobiellen Probe mit einem Enzymcocktail, umfassend eine Proteinase und eine Glycosidhydrolase, um ein Lysat der mikrobiellen Probe herzustellen; und
c) Aufreinigen der mikrobiellen gDNA aus dem Lysat der mikrobiellen Probe.

2. Verfahren nach Anspruch 1, wobei der mechanische Aufschluss die Verwendung von Beads umfasst.

3. Verfahren nach Anspruch 2, wobei die Beads
(a) Granatbeads, Carbidbeads, Metallbeads, Glasbeads oder Keramikbeads sind;
(b) einen Durchmesser zwischen 0,1 mm und
3,0 mm aufweisen; oder
(c) einen Durchmesser zwischen 0,1 mm und
0,5 mm aufweisen.

4. Verfahren nach Anspruch 1, wobei das mechanische Aufschließen die Verwendung eines Schüttelhomogenisators umfasst.

5. Verfahren nach Anspruch 1, wobei sich die mikrobielle Probe, mit der der mechanischen Aufschluss durchgeführt wird, in einem Lysepuffer befindet, und wobei der Lysepuffer ein kationisches Detergens umfasst.

6. Verfahren nach Anspruch 1, wobei die mechanisch aufgeschlossene mikrobielle Probe mit dem Enzymcocktail für etwa 30 bis 60 Minuten oder zwischen etwa 25°C und etwa 60°C in Kontakt gebracht wird.

7. Verfahren nach Anspruch 1, wobei die Proteinase eine Serinprotease ist.

8. Verfahren nach Anspruch 1, wobei die Proteinase in einer Konzentration zwischen etwa 0,1 und etwa 1,0 mg/ml vorliegt.

9. Verfahren nach Anspruch 1, wobei die Glycosidhydrolase eine N-Acetyl-Muramidase, N-Acetyl-Muramid-Glycanhydrolase oder Muramidase ist.

10. Verfahren nach Anspruch 1, wobei der Enzymcocktail ferner eine zweite Glycosidhydrolase umfasst, wobei die Glycosidhydrolase von der zweiten Glycosidhydrolase verschieden ist.

11. Verfahren nach Anspruch 1, wobei der Enzymcocktail zusätzliche Enzyme umfasst, die aus der Gruppe ausgewählt sind bestehend aus RNase A, Lysostephin, Labiase, Lysyl-Endopeptidase und einer Kombination davon.

12. Verfahren nach Anspruch 1, wobei die Aufreinigung der mikrobiellen gDNA aus dem Lysat Folgendes umfasst:
1) Zugeben eines Bindungspuffers, umfassend ein chaotropes Mittel und einen Chelatbildner, zu dem Lysat der mikrobiellen Probe, um eine Bindungsmischung herzustellen;
2) Filtrieren der Bindungsmischung über eine DNA-Bindungsmatrix;
3) Filtrieren eines Waschpuffers, umfassend einen Alkohol, über die Matrix; und
4) Filtrieren eines Elutionspuffers über die Matrix, um ein Eluat herzustellen, wobei das Eluat die mikrobielle gDNA umfasst.

13. Verfahren nach Anspruch 1, wobei die mikrobielle Probe Gram-negative Bakterien, Gram-positive Bakterien oder eine Kombination von beiden umfasst.

14. Verfahren nach Anspruch 1, wobei die mikrobielle Probe Umweltisolate umfasst.

## Revendications

1. Procédé pour l'extraction d'ADN génomique (ADNg) à partir d'un échantillon microbien comprenant les étapes de :
a) soumission de l'échantillon microbien à une perturbation mécanique ;
b) à la suite de l'étape (a) mise en contact de l'échantillon microbien perturbé de manière mécanique avec un cocktail d'enzymes comprenant une protéinase et une glycoside hydrolase pour produire un lysat de l'échantillon microbien ; et
c) purification d'ADNg microbien du lysat de l'échantillon microbien.

2. Procédé selon la revendication 1, la perturbation mécanique comprenant l'utilisation de billes.

3. Procédé selon la revendication 2, les billes
(a) étant des billes de grenat, des billes de carbure, des billes métalliques, des billes de verre, ou des billes de céramique ;
(b) possédant un diamètre compris entre 0,1 mm et 3,0 mm ; ou
(c) possédant un diamètre compris entre 0,1 mm et 0,5 mm.

4. Procédé selon la revendication 1, la perturbation mécanique comprenant l'utilisation d'un homogénéisateur agitateur.

5. Procédé selon la revendication 1, l'échantillon microbien soumis à une perturbation mécanique étant dans un tampon de lyse, et le tampon de lyse comprenant un détergent cationique.

6. Procédé selon la revendication 1, l'échantillon microbien perturbé de manière mécanique étant mis en contact avec le cocktail d'enzymes pendant environ 30 à 60 minutes ou entre environ 25 °C et environ 60 °C.

7. Procédé selon la revendication 1, la protéinase étant une sérine protéase.

8. Procédé selon la revendication 1, la protéinase étant à une concentration d'entre environ 0,1 et environ 1,0 mg/mL.

9. Procédé selon la revendication 1, la glycoside hydrolase étant une N-acétyl-muramidase, une N-acétylmuramide glycanhydrolase, ou une muramidase.

10. Procédé selon la revendication 1, le cocktail d'enzymes comprenant en outre une deuxième glycoside hydrolase, la glycoside hydrolase étant différente de la deuxième glycoside hydrolase.

11. Procédé selon la revendication 1, le cocktail d'enzymes comprenant des enzymes supplémentaires choisies dans le groupe constitué par une RNase A, la lysostephine, la labiase, la lysyl-endopeptidase, et une combinaison correspondante.

12. Procédé selon la revendication 1, la purification d'ADNg microbien du lysat comprenant :
1) l'ajout d'un tampon de liaison comprenant un agent chaotropique et un agent chélatant au lysat de l'échantillon microbien pour produire un mélange liant ;
2) la filtration du mélange liant à travers une matrice de liaison d'ADN ;
3) la filtration d'un tampon de lavage, comprenant un alcool, à travers la matrice ; et
4) la filtration d'un tampon d'élution à travers la matrice pour produire un éluat, l'éluat comprenant l'ADNg microbien.

13. Procédé selon la revendication 1, l'échantillon microbien comprenant des bactéries Gram-négatives, des bactéries Gram-positives, ou une combinaison des deux.

14. Procédé selon la revendication 1, l'échantillon microbien comprenant des isolats environnementaux.
